# EUROPÄISCHE PATENTSCHRIFT

(11) **EP 1 853 267 B1**
(45) Veröffentlichungstag und Bekanntmachung des Hinweises auf die Patenterteilung: **23.01.2013**
(21) Anmeldenummer: 06723165.4
(22) Anmeldetag: 01.03.2006
(51) Int. Cl.: A61K 31/525, A61K 31/4415, A61K 31/714, A61P 9/10, A61P 7/02

(54) **MITTEL, ENTHALTEND FOLSÄURE, VITAMIN B6 UND VITAMIN B12, UND DESSEN VERWENDUNG**
FOLIC ACID-, VITAMIN B6- AND VITAMIN B12-CONTAINING AGENTS AND THE USE THEREOF
PRODUITS CONTENANT DE L'ACIDE FOLIQUE, DE LA VITAMINE B6, ET DE LA VITAMINE B12, ET LEUR UTILISATION

(30) Priorität: 01.03.2005 DE 102005009379
(43) Veröffentlichungstag der Anmeldung: 14.11.2007
(73) Patentinhaber: PHRONTIER S.A.R.L., 76490 Caudebec-en-Caux (FR)
(72) Erfinder: GÖRNE, Martin, 22337 Hamburg (DE)
(74) Vertreter: Reitstötter - Kinzebach
(86) Internationale Anmeldenummer: PCT/EP2006/001875
(87) Internationale Veröffentlichungsnummer: WO 2006/092294

(56) Entgegenhaltungen:
- EP-A- 0 595 005
- WO-A-94/06415
- WO-A-03/068231
- US-A1- 2003 225 030
- US-B1- 6 297 224
- NAURATH HANS J ET AL: "Effects of vitamin B12, folate, and vitamin B6 supplements in elderly people with normal serum vitamin concentrations" LANCET, LITTLE, BROWM AND CO., BOSTON,, US, Bd. 346, Nr. 8967, 1995, Seiten 85-89, XP002183709 ISSN: 0099-5355

## Beschreibung

Die vorliegende Erfindung betrifft Mittel, enthaltend Folsäure, Vitamin B6 und Vitamin B12, und deren Verwendung zur Regulation von Homocysteinspiegeln. Die Mittel sind damit vor allem zur vorbeugenden und akuten Behandlung von vaskulären Erkrankungen brauchbar. Beschrieben werden insbesondere pharmazeutische Mittel und Nahrungsergänzungsmittel mit einer entsprechenden Wirkstoffkombination sowie Mittel in Form von Handelspackungen mit entsprechenden Kombinationspräparaten oder Monopräparaten zur kombinierten Anwendung.

Seit kurzem ist bekannt, dass Homocystein ein Risikofaktor für koronare, periphere und zerebrale vaskuläre Erkrankungen darstellt. Patienten mit hereditärer Hyperhomocysteinämie, einer autosomal rezessiven Erkrankung, haben Homocystein-Plasmaspiegel, die etwa 10-20-fach über den Normalwerten liegen. Bei Kindern, die von der homozygoten Form der Erkrankung betroffen sind, machen sich frühzeitig Gefäßveränderungen bemerkbar, welche die hauptsächliche Ursache für den häufig lethalen Ausgang der Erkrankung im Kindesalter darstellen. Während die erhöhten Homocysteinspiegel bei den genetisch bedingten Formen der Hyperhomocysteinämie in der Regel auf einen Mangel an Cystathionin-β-Synthase und/oder auf eine Mutation der 5,10-Methylentetrahydrofolat-Reduktase zurückzuführen sind, können auch Veränderungen des Folsäure-, Vitamin B6-, Vitamin B12- und Betainstoffwechsels zu erhöhten Homocysteinspiegeln führen. Dementsprechend können auch erworbene Formen der Hyperhomocysteinämie auftreten. Beispielsweise können Niereninsuffizienzen oder ein Mangel an Folsäure, Cobalamin und/oder Pyridoxin bzw. Metaboliten davon zu erhöhten Homocysteinspiegeln führen. Insbesondere bei älteren Menschen gilt ein derartiger Vitaminmangel, der durch eine unzureichende Zufuhr oder durch Malabsorption verursacht werden kann, als die häufigste Ursache von erworbener Hyperhomocysteinämie.

Vitamin B12 ist erforderlich, um eine 1-Kohlenstoffeinheit von Folsäure auf Homocystein zu übertragen und dieses in Methionin zu überführen. Vitamin B6 ist an einem weiteren Stoffwechselweg für den Abbau von überschüssigem Homocystein beteiligt.

Es wurde bereits vorgeschlagen, verschiedenste, Folsäure, Vitamin B6 und Vitamin B12 enthaltende Vitaminpräparate zur Senkung erhöhter Homocysteinspiegel einzusetzen.

Beispielsweise beschreibt die US 5,932,624 ein Mittel, das 500 µg Folsäure, 25 µg Vitamin B12 und 10 mg Vitamin B6 enthält. In Abhängigkeit vom Patientenzustand sollen im Allgemeinen 300 bis 2000 µg Folsäure, 25 bis 1000 µg Vitamin B12 und 5 bis 20 mg Vitamin B6 so verabreicht werden, dass die Homocystein-Plasmaspiegel auf Normalwerte sinken.

Die in US 6,274,170 angegebene Kombination aus Vitaminen und Aspirin zur Behandlung von atherosklerotischen kardiovaskulären Erkrankungen enthält 400 bis 1000 µg Folsäure, 3 bis 25 mg Vitamin B6 und 5 bis 500 µg Vitamin B12.

Ein Multivitamin- und Mineral-Supplement, der neben einer Reihe weiterer Vitamine und essentieller Spurenelemente 800 µg Folsäure, 25 mg Vitamin B6 und 400 µg Vitamin B12 enthält, wird in der US 6,299,896 beschrieben. Auch dieses Mittel soll die Homocysteinspiegel senken können.

Eine tägliche Aufnahme von 180 bis 800 µg Folsäure, 1,6 bis 4,6 mg Vitamin B6 und 1,5 bis 4,0 µg Vitamin B12 zusammen mit β-Glukan- oder Glukomannan-haltigen Fasern wird in der US 6,210,686 empfohlen, um die Zusammensetzung an Serumlipiden zu verbessern, Homocysteinspiegel zu senken und Lipoproteine vor Oxidation zu schützen.

Zur Vorbeugung und Behandlung erhöhter Homocystein-, Cystathionin-, Methylmalonsäure- oder 2-Methylcitronsäure-Serumspiegel sollen gemäß US 6,297,224 und den dazu verwandten US 6,207,651 und US 5,563,126 Vitaminzubereitungen eingesetzt werden, die 0,4 mg oder 1,0 mg Folsäure zusammen mit 25 mg Vitamin B6 und 2,0 mg Vitamin B12 enthalten.

US 6,129,918 beschreibt ein Mittel auf Knoblauch-Basis zur Senkung von Homocystein-Plasmaspiegeln. Zusätzlich zu Knoblauch oder Knoblauchextrakt kann dieses Mittel Folsäure, Vitamin B6 und Vitamin B12 enthalten. Die drei zuletzt genannten Wirkstoffe sollen dabei die vorteilhaften Eigenschaften des Knoblauchs verstärken.

WO 03/068231 betrifft Mittel, die Folsäure, Vitamin B6 und Vitamin B12 enthalten, sowie deren Verwendung zur Regulation von Homocysteinspiegeln. Das Gewichtsmengenverhältnis von Folsäure zu Vitamin B6 und von Vitamin B12 zu Vitamin B6 wird in einem Bereich von etwa 1:67 bis 1:150 und das Gewichtsmengenverhältnis von Folsäure zu Vitamin B12 in einem Bereich von etwa 1:0,67 bis 1:1,50 angegeben. Diese Mittel besitzen eine sichere Homocysteinspiegel senkende Wirkung. Allerdings stellte man fest, dass bei einigen, auf diese Weise behandelten Patienten unerwünschte Nebenwirkungen, wie Parästhesien an den Extermitäten, auftraten.

Es wurde nun überraschenderweise gefunden, dass derartige Nebenwirkungen vermieden und Homocysteinspiegel dennoch wirksam gesenkt werden können, wenn man anstatt der aus WO 03/068231 bekannten Kombination aus Folsäure, Vitamin B6 und Vitamin B12 eine in der Vitamin B6-Menge reduzierte Kombination einsetzte.

Gegenstand der vorliegenden Erfindung sind daher Mittel auf Basis von Folsäure, Vitamin B6 und Vitamin B12 beziehungsweise physiologisch akzeptabler Derivate und/oder Salze davon, die dadurch gekennzeichnet sind, dass das Mengenverhältniss von Folsäure zu Vitamin B6 und von Vitamin B12 zu Vitamin B6 in einem Bereich von etwa 1:41 etwa 1:61 und das Mengenverhältniss von Folsäure zu Vitamin B12 in einem Bereich von etwa 1:0,82 etwa 1:1,22 liegt.

Die erfindungsgemäßen Mittel auf Basis von Folsäure, physiologisch akzeptabler Derivate oder Salze davon (zwecks Vereinfachung auch als "Folsäuren" oder "Folsäure-Komponente" bezeichnet), Vitamin B6, physiologisch akzeptabler Derivate oder Salze davon (zwecks Vereinfachung auch als "B6-Vitamine" oder "Vitamin B6-Komponente" bezeichnet) und Vitamin B12, physiologisch akzeptabler Derivate oder Salze davon (zwecks Vereinfachung auch als "B12-Vitamine" oder "Vitamin B12-Komponente" bezeichnet) bieten wesentliche Vorteile bei der Regulation von Homocysteinspiegeln und damit bei der vorbeugenden und akuten Behandlung von vaskulären Erkrankungen.

Gegenstand der vorliegenden Erfindung ist daher auch die Verwendung der erfindungsgemäßen Kombination aus Folsäure, Vitamin B6 und Vitamin B12 beziehungsweise physiologisch akzeptablen Derivaten und/oder Salzen davon zur Regulation des Homocysteinspiegels. Die Regulation betrifft insbesondere im akuten Bereich die Senkung erhöhter Homocysteinspiegel, d.h. insbesondere die Behandlung von Hyperhomocysteinämie, sowie im prophylaktischen Bereich die Vermeidung erhöhter Homocysteinspiegel und die Beibehaltung normaler Homocysteinspiegel. Mit der Regulation von Homocysteinspiegeln verbunden ist insbesondere eine prophylaktische Behandlung von Erkrankungen, die mit erhöhten Homocysteinspiegeln in Zusammenhang stehen, also insbesondere solche, die von erhöhten Homocysteinspiegeln begleitet oder versucht werden.

Ein weiterer Gegenstand der vorliegenden Erfindung ist daher die Verwendung der erfindungsgemäßen Kombination aus Folsäure, Vitamin B6 und Vitamin B12 beziehungsweise physiologisch akzeptablen Derivaten und/oder Salzen davon zur Behandlung von Erkrankungen, die mit einem erhöhten Homocysteinspiegel in Zusammenhang stehen. Hierzu gehören insbesondere vaskuläre Erkrankungen, wie Arteriosklerose, venöse Thrombosen und arterielle Verschlüsse, foetale Schäden, wie Neuralrohrdefekte, und neurodegenerative Erkrankungen, wie bestimmte Formen der Alzheimerschen Demenz.

In diesem Sinne sind Gegenstand der Erfindung auch Mittel zur Regulation des Homocysteinspiegels und zur Behandlung von Erkrankungen, die mit erhöhten Homocysteinspiegeln in Zusammenhang stehen. Diese Mittel basieren auf der erfindungsgemäßen Wirkstoffkombination und gegebenenfalls weiteren Wirkstoffen, wobei die Wirkstoffe bzw. Wirkstoffkomponenten gemeinsam in einer Formulierung oder getrennt in wenigstens zwei oder drei verschiedenen Formulierungen formuliert sein können.

Besondere Vorteile einer Anwendung der erfindungsgemäßen Wirkstoffkombination zeigen sich bei bestimmten Patientengruppen, bei denen die Behandlung mit Homocysteinspiegel senkenden Mitteln mit Nebenwirkungen verbunden ist. Hierzu gehören insbesondere Parästhesien im Bereich der Extermitäten, insbesondere an Hand, Arm, Fuss und/oder Bein, die mit der Verabreichung einer relativ hohen Vitamin B6-Menge in Zusammenhang zu stehen scheinen. Deshalb ist es erfindungsgemäß bevorzugt, das Mengenverhältniss von Folsäure und/oder Vitamin B12 zu Vitamin B6 auf höchstens 1:61 zu begrenzen (d. h. die Menge an Vitamin B6 beträgt höchstens das 61-fache der Menge an Folsäure bzw. Vitamin B12). Dies gilt insbesondere dann, wenn die Tagesdosis von Folsäure bzw. Vitamin B12 mit jeweils bis zu 1,2 mg relativ hoch gewählt wird. Eine weitere, davon unabhängige Möglichkeit besteht darin, die Tageshöchstdosis von Vitamin B6 auf weniger als 70 mg zu begrenzen.

Von besonderem Vorteil sind Mittel und Verwendungen, bei denen das Mengenverhältniss von Folsäure zu Vitamin B6 zu Vitamin B12 etwa 1 zu 50 zu 1 beträgt.

Einem besonderen Aspekt der Erfindung zufolge, erfolgt die Verwendung der Wirkstoffe in den oben angegebenen Mengenverhältnissen derart, dass einem zu behandelnden Individuum mit dem Gewicht eines durchschnittlichen Erwachsenen von etwa 75 kg vorzugsweise eine Tagesdosis von weniger als 70 mg Vitamin B6, insbesondere von weniger als 65 mg Vitamin B6 und vor allem von höchstens 60 mg Vitamin B6 verabreicht wird.

Dabei beziehen sich die angegebenen Mengenverhältnisse auf Gewichtsmengen der Wirkstoffe Folsäure, Vitamin B6 und Vitamin B12, so daß für Salze und Derivate erforderlichenfalls eine entsprechende Umrechnung zu erfolgen hat. Dies gilt analog für die in der vorliegenden Beschreibung angegebene Wirkstoffanteile. Alternativ kann man die Verhältnisse auch auf molare Mengen beziehen, so dass unter der Annahme, dass 1 Mol des betreffenden Derivats oder Salzes 1 Mol Folsäure, Vitamin B6 oder Vitamin B12 enthält, die Molmengenverhältnisse für Folsäure, Vitamin B6, Vitamin B12 und deren Derivate und/oder Salze einheitlich ausgedrückt werden können.

"Folsäure" bezeichnet erfindungsgemäß N-Pteroylglutaminsäure der Formel I 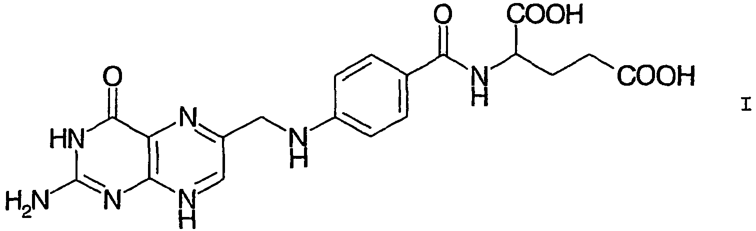 die unter diese Formel fallenden optischen Isomere sowohl als Gemische, z.B. als Racemat, als auch in Reinform, z.B. R- oder S-Enantiomere, eingeschlossen. Bevorzugt ist N-Pteroyl-L-glutaminsäure der Formel Ia

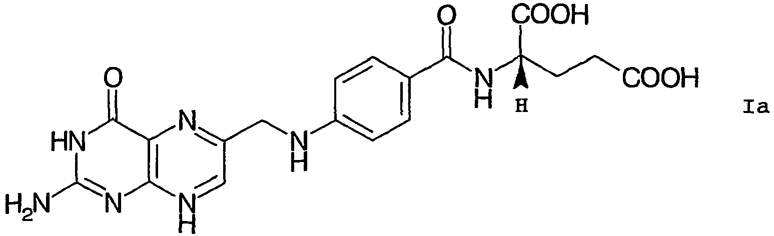

Zu den Folsäure-Derivaten gehören vor allem Folsäure-Metabolite sowie Amide und Ester der Folsäure wie auch der Metabolite. Vorteilhaft sind Amide und Ester, die unter physiologischen Bedingungen hydrolisierbar sind, wie Amide mit C₁-C₁₀-Alkylaminen oder Ester mit C₁-C₁₀-Alkoholen. Eine besondere Form der Amide sind N-Pteroylpolyglutaminsäuren.

Zu den Folsäure-Metaboliten gehören vor allem H₄-Folsäuren der Formel Ib 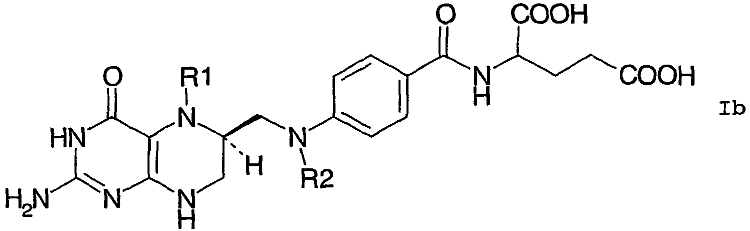 worin R1 für Wasserstoff, Methyl, -HC=O (Formyl) oder -HC=NH (Formimino) steht und R2 für Wasserstoff oder -HC=O (Formyl) steht, oder R1 und R2 zusammen genommen eine Methylen oder Methenylbrücke bilden. Die unter diese Formel fallenden optischen Isomere sind obigen Ausführungen entsprechend eingeschlossen, wobei auch hier die L-Glutaminsäure-Derivate bevorzugt sind. Insbesondere sind Tetrahydrofolsäure, 5-Methyltetrahydrofolsäure, 5,10-Methylentetrahydrofolsäure, 5-Formyltetrahydrofolsäure, 10-Formyltetrahydrofolsäure, 5-Formiminotetrahydrofolsäure und 5,10-Methenyltetrahydrofolsäure zu nennen.

Zu physiologisch akzeptablen Salzen von Folsäure bzw. Folsäure-Derivaten gehören Säure- und Basenadditionssalze sowie entsprechende Mischformen.

Zu den Säureadditionssalzen zählen Salze von Folsäure bzw. Folsäure-Derivaten mit anorganischen Säuren, wie Salzsäure, Schwefelsäure, Salpetersäure oder Phosphorsäure, oder organischen Säuren, insbesondere Carbonsäuren, z.B. Essigsäure, Weinsäure, Milchsäure, Citronensäure, Apfelsäure, Mandelsäure, Ascorbinsäure, Maleinsäure, Fumarsäure, Gluconsäure oder Sulfonsäuren, z. B. Methansulfonsäure, Benzolsulfonsäure und Toluolsulfonsäure, und dergleichen.

Zu den Basenadditionssalzen zählen Salze von Folsäure bzw. Folsäure-Derivaten mit anorganischen Basen, beispielsweise Metallhydroxiden bzw. -carbonaten von Alkali-, Erdalkali- oder Übergangsmetallen, oder mit organischen Basen, beispielsweise Ammoniak oder basischen Aminosäuren, wie Arginin und Lysin, Aminen, z.B. Methylamin, Dimethylamin, Trimethylamin, Triethylamin, Ethylamin, Diethylamin, Ethylendiamin, Ethanolamin, Diethanolamin, 1-Amino-2-propanol, 3-Amino-1-propanol oder Hexamethylentetramin, gesättigten cyclischen Aminen mit 4 bis 6 Ringkohlenstoffatomen, wie Piperidin, Piperazin, Pyrrolidin und Morpholin, sowie weiteren organischen Basen, beispielsweise N-Methylglucamin, Kreatin und Tromethamin, sowie quaternären Ammoniumverbindungen, wie Tetramethylammonium und dergleichen.

Bevorzugt sind Salze mit anorganischen Basen, z.B. Na-, K-, Mg-, Ca-, Zn-, Cr- und Fe-Folate.

"Vitamin B6" bezeichnet erfindungsgemäß 4,5-Bis(Hydroxymethyl)-2-methyl-3-pyridinol der Formel II 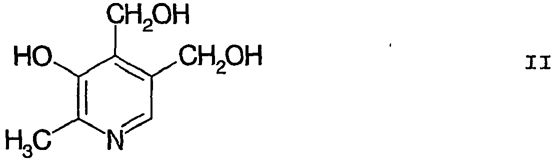 auch als Pyridoxin (INN) bezeichnet.

Zu den Vitamin B6-Derivaten gehören vor allem Pyridoxale und Pyridoxamine sowie Ester der Pyridoxine, Pyridoxale und Pyridoxamine. Vorteilhaft sind auch hier Ester, die unter physiologischen Bedingungen hydrolisierbar sind.

Insbesondere zu nennen sind in diesem Zusammenhang die Pyridoxine, Pyridoxale und Pyridoxamine der Formel IIa 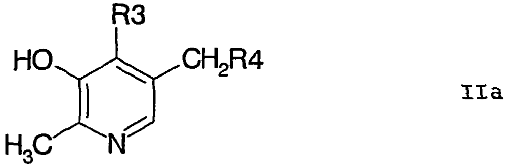 worin R3 für CH₂OH, CHO oder CH₂NH₂ steht und R4 für OH oder OPO₃H₂ steht.

Zu physiologisch akzeptablen Salzen von Vitamin B6 bzw. Vitamin B6-Derivaten gehören insbesondere Säureadditionssalze, z.B. mit den oben genannten anorganischen und organischen Säuren. Insbesondere ist das Hydrochlorid, vor allem Pyridoxin-HCl, zu nennen.

"Vitamin B12" wird auch als Cyanocobalamin oder Cobalamin bezeichnet.

Zu den Vitamin-B12-Derivaten gehören insbesondere Cobalamine, in denen die Cyanogruppe des Cyanocobalamins durch andere Koordinationspartner des Cobalts ersetzt ist. Hierzu zählen vor allem das Hydroxocobalamin, Aquocobalamin, Nitrosocobalamin, Methylcobalamin und Adenosylcobalamin (Coenzym B12).

Zu physiologisch akzeptablen Salzen von Vitamin B12 bzw. Vitamin B12-Derivaten gehören insbesondere Säureadditionssalze z.B. mit den oben genannten anorganischen und organischen Säuren. Insbesondere ist das Acetat des Hydoxocobalamins zu nennen.

Folsäuren, B6- und B12-Vitamine sind hinlänglich bekannt und können entweder bezogen oder in an sich bekannter Weise zur Verfügung gestellt werden.

Neben den Folsäure-, Vitamin B6- und Vitamin-B12-Komponenten können die erfindungsgemäßen Mittel weitere Wirkstoffe miteinbeziehen. Bei diesen Wirkstoffen kann es sich insbesondere um solche handeln, deren Wirkung der Folsäure-, Vitamin B6- bzw. Vitamin B12-vermittelten Wirkung ähnlich ist oder diese ergänzt und die insbesondere den erfindungesgemäßen Verwendungszwecken entspricht. So kann es von Vorteil sein, zusätzlich zur erfindungsgemäßen Kombination, Homocysteinspiegel senkende Wirkstoffe, Antithrombotika, Antisklerotika und ähnliches zu verabreichen.

Assays zur Bestimmung von Homocysteinspiegeln, die normalerweise in einem Bereich von 5 bis 15 µmol/l Blutplasma liegen, sind bekannt (vgl. z.B. den eingangs geschilderten Stand der Technik).

Erhöhte Homocysteinspiegel werden als Hyperhomocysteinämie bezeichnet. Mithilfe der erfindungsgemäßen Mittel können erhöhte Homocysteinspiegel gesenkt bzw. präventiv vermieden werden.

Je nach Homocysteinspiegel, werden Hyperhomocysteinämien in drei Klassen eingeteilt:

Leichte Hyperhomocysteinämien sind gekennzeichnet durch Homocysteinspiegel in einem Bereich von mehr als 15 und bis zu 30 µmol/1 Blutplasma.

Mittlere Hyperhomocysteinämien sind gekennzeichnet durch Homocysteinspiegel in einem Bereich von mehr als 30 und bis zu 100 µmol/l Blutplasma.

Hohe Hyperhomocysteinämien sind gekennzeichnet durch Homocysteinspiegel von mehr als 100 µmol/l Blutplasma.

Besondere Vorteile ergeben sich erfindungsgemäß im Hinblick auf die Behandlung von mittleren Hyperhomocysteinämien.

Die vorliegende Erfindung richtet sich insbesondere auf die Behandlung eines oder mehrerer der folgenden Krankheitsbilder:

Hereditäre Hyperhomocysteinämie. Das Krankheitsbild der hereditären Hyperhomocysteinämie ist gekennzeichnet durch genetisch bedingte Störungen des Homocystein-Stoffwechsels. Zu derartigen Stoffwechselstörungen gehören insbesondere ein Fehlen (homozygote Form) oder Mangel (heterozygote Form) der Cystathionin-β-Synthase, ein Mangel an Methylentetrahydrofolat-Reduktase, eine mutationsbedingte Veränderung der Methylentetrahydrofolat-Reduktase zu einem thermolabilen Derivat davon sowie eine Reihe weiterer Veränderungen des Folsäure-, Vitamin B6-, Vitamin B12- und des Betainstoffwechsels. Zu klinischen Symptomen einer hereditären Hyperhomocysteinämie gehören Homocysteinurie, geistige Retardierung, Subluxation der Augenlinsen, Skelettanomalien und/oder vaskuläre Erkrankungen, die damit als Symptom oder Syndrom erfindungsgemäß akut oder präventiv behandelbar sind.

Erworbene Hyperhomocysteinämie. Erworbene Formen der Hyperhomocysteinämie sind in der Regel gekennzeichnet durch Mangelerscheinungen, die zu einer Akkumulation von Homocystein führen. Beispielsweise können Mängel an Folsäure und Folsäurederivaten, Vitamin B12 und Vitamin B12-Derivaten, Vitamin B6 und Vitamin B6-Derivaten sowie ein allgemeiner Vitaminmangel, zu erhöhten Homocysteinspiegeln führen. Dabei kann der Vitaminmangel beispielsweise durch eine unzureichende Zufuhr oder durch Malabsorption des oder der jeweiligen Vitamine bedingt sein. Auch können Medikamente, die den Folsäure-Metabolismus beeinflussen können, wie Methotrexat oder Antikonvulsiva; die den Vitamin B12-Metabolismus beeinflussen können, wie Nitrokörper; oder die den Vitamin-B6-Metabolismus beeinflussen können, wie Theophyllin, erhöhte Homocysteinspiegel verursachen. Ferner beeinflussen Alter, Geschlecht, Zigarettenrauchen, arterielle Hypertonie, Hypercholesterinämie und Bewegungsmangel den Homocystein-Plasmaspiegel.

Weiterhin richtet sich die vorliegende Erfindung auf die Behandlung von Erkrankungen, die mit erhöhten Homocysteinspiegeln in Zusammenhang stehen, insbesondere damit einhergehen oder dadurch verursacht werden. Hierzu zählen vor allem vaskuläre Erkrankungen, foetale Mißbildungen und bestimmte neurodegenerative Erkrankungen. In diesem Indikationsbereich ist vor allem die Prävention von Bedeutung.

Unter vaskulären Erkrankungen versteht man Erkrankungen der peripheren, koronaren und zerebralen Gefäße. Insbesondere zu nennen sind Veränderungen an den Gefäßendothelzellen, eine Proliferation der Muskelzellen und/oder eine Verdickung der Gefäßintima. Erfindungsgemäß behandelbar sind damit insbesondere Arteriosklerosen, venöse Thrombosen, arterielle Verschlüsse und weitere arteriovenöse Gefäßleiden.

Foetale Mißbildungen, insbesondere Neuralrohrdefekte, können im Rahmen einer Schwangerschaft auftreten, wenn die Mutter unter erhöhten Homocysteinspiegeln leidet.

Erhöhte Homocysteinspiegel können auch an neurodegenerativen Erkrankungen, insbesondere vaskulär bedingten Formen der Altersdemenz, beteiligt sein.

Somit richtet sich die Erfindung einem besonderen Aspekt zufolge auf Verminderung des Risikos für das Auftreten der vorstehend beschriebenen vaskulären Erkrankungen, foetaler Mißbildungen und der neurodegenerativen Erkrankungen.

Besondere Vorteile einer Anwendung der erfindungsgemäßen Wirkstoffkombination zeigen sich bei bestimmten Patientengruppen, bei denen die Verabreichung von Folsäure, Vitamin B6 und Vitamin B12 zur Senkung des Homocysteinwertes Nebenwirkungen auslösen kann. Hierzu gehören insbesondere solche Patienten, bei denen Parästhesien im Bereich der Extermitäten, insbesondere an Hand, Arm, Fuss und/oder Bein, auftreten. Solche Nebenwirkungen scheinen insbesondere dann häufiger aufzutreten, wenn die Vitamin B6-Tagesdosis mehr als 70 mg, 80 mg, 90 mg, oder gar 100 mg beträgt.

Die erfindungsgemäßen Mittel und Anwendungen gewinnen bei Erwachsenen mit zunehmendem Lebensalter an Bedeutung. In der Gruppe der über 40-jährigen und vor allem der über 50-jährigen bringt die Behandlung besondere Vorteile mit sich. Die erfindungsgemäße Behandlung ist insbesondere dann angezeigt, wenn Hinweise auf Arteriosklerosen, arterielle Verschlüsse, venöse Thrombosen und/oder vaskulär bedingte Formen der Altersdemenz vorliegen oder ein Risiko für diese Erkrankungen besteht. Eine weitere Gruppe, bei denen die erfindungsgemäße Behandlung besondere Vorteile mit sich bringen kann, sind Kinder mit hereditärer Hyperhomocysteinämie sowie schwangere Frauen, selbst wenn Hinweise auf vaskuläre Erkrankungen nicht vorliegen und die Homocysteinspiegel nur leicht erhöht sind.

Erfindungsgemäß wird dem zu behandelnden Individuum, vorzugsweise einem Säuger, insbesondere einem Menschen und auch einem Nutz- oder Haustier, eine wirksame Menge der erfindungesgemäßen Wirkstoffkombination aus Folsäure-Komponente, Vitamin B6-Komponente und Vitamin B12-Komponente, in der Regel der pharmazeutischen, tierarzneilichen oder lebensmitteltechnologischen Praxis entsprechend formuliert, verabreicht. Wirksam ist eine Menge erfindungsgemäß insbesondere dann, wenn sie eine signifkante Senkung des Homocysteinspiegels, vorteilhafterweise bis in den Normalbereich, bewirkt.

Die Behandlung erfolgt in der Regel durch einmaliges oder mehrmaliges tägliches Verabreichen einer geeigneten Dosis gegebenenfalls zusammen oder im Wechsel mit anderen Wirkstoffen oder wirkstoffhaltigen Präparaten, so dass einem zu behandelnden Individuum mit dem Gewicht eines durchschnittlichen Erwachsenen von etwa 75 kg in der Regel eine Tagesmindestdosis von etwa 0,8 mg, vorzugsweise etwa 0,9 mg und vorteilhafterweise etwa 1 mg Folsäure, von etwa 40 mg, vorzugsweise etwa 45 mg und vorteilhafterweise etwa 50 mg Vitamin B6, sowie von etwa 0,8 mg, vorzugsweise etwa 0,9 mg und vorteilhafterweise etwa 1 mg Vitamin B12 verabreicht wird. Einem anderen Aspekt zufolge beträgt die Tageshöchstdosis in der Regel etwa 1,2 mg, vorzugsweise etwa 1,1 mg und vorteilhafterweise etwa 1 mg Folsäure, etwa 60 mg, vorzugsweise etwa 55 mg und vorteilhafterweise etwa 50 mg Vitamin B6, sowie etwa 1,2 mg, vorzugsweise etwa 1,1 mg und vorteilhafterweise etwa 1 mg Vitamin B12. Bei Abweichungen vom Durchschnittsgewicht sollte die Tagesdosis entsprechend angepaßt werden. Diese Anpassung erfolgt in üblicher Weise durch den Fachmann erforderlichenfalls unter Berücksichtigung analytischer Kontrolluntersuchungen. Weiterhin können sich Abweichungen in der Tagesdosis durch den verordnenden Arzt auch aufgrund des Gesundheitszustandes des zu behandlenden Individuums ergeben.

Die Behandlung erfolgt in der Regel über einen angemessenen Zeitraum im Bereich von Tagen oder Wochen. Zweckmäßig ist eine Normalisierung der Homocysteinspiegel innerhalb eines Behandlungszeitraums von etwa 1 bis 4 Wochen. Erforderlichenfalls wird die Behandlung auch nach Normalisierung der Homocysteinspiegel fortgesetzt. Dies gilt insbesondere für hereditäre Formen der Hyperhomocysteinämie und erworbene Formen, bei denen eine ursächliche Behandlung nicht möglich ist oder keinen Erfolg gezeigt hat und das Absetzen der erfindungsgemäßen Behandlung ein erneutes Ansteigen der Homocysteinspiegel zur Folge hätte.

Die Erfindung betrifft auch die Herstellung von Mitteln zur Behandlung eines Individuums, vorzugsweise eines Säugers, insbesondere eines Menschen und auch eines Nutz- oder Haustieres.

Zu den Mitteln gehören insbesondere pharmazeutische Mittel, Nahrungsergänzungsmittel und Lebensmittel, z.B. funktionale oder diätetische Lebensmittel. Die erfindungsgemäßen Lebensmittel besitzen neben einer vorwiegend nährwertbezogenen Funktion zusätzlich eine wirkstoffbezogene Funktion, welche insbesondere die erfindungsgemäße Wirkstoffkombination betrifft. Sie werden daher als funktionale oder diätetische Lebens- oder Nahrungsmittel bezeichnet. Nahrungsergänzungsmittel dienen zur Ergänzung der täglichen Ernährung mit der erfindungsgemäßen Wirkstoffkombination, wobei die nährwertbezogene Funktion des Nahrungsergänzungsmittels für sich genommen in den Hintergrund tritt.

Einem Aspekt zufolge betrifft die vorliegende Erfindung Formulierungen, enthaltend
i) wenigstens einen Wirkstoff aus der Folsäure-Gruppe (Folsäure, physiologisch akzeptable Derivate und/oder Salze davon),
ii) wenigstens einen Wirkstoff aus der Vitamin B6-Gruppe (Vitamin B6, physiologisch akzeptable Derivate und/oder Salze davon), und
iii) wenigstens einen Wirkstoff aus der Vitamin B12-Gruppe (Vitamin B12, physiologisch akzeptable Derivate und/oder Salze davon), sowie
gegebenenfalls wenigstens einen weiteren Wirkstoff und eine Formulierungsgrundlage, in den erfindungsgemäß angegebenen Mengenverhältnissen.

Somit umfaßt die Wirkstoffkombination im Sinne der Erfindung als Wirkstoffkomponente i) Folsäure, ein physiologisch akzeptables Derivat und/oder Salz davon. Gemische dieser Formen sind möglich, jedoch nur in bestimmten Fällen in Betracht zu ziehen. Gemäß einer besonderen Ausführungsform besteht die Wirkstoffkomponente i) aus wenigstens 90 Gew.-% Folsäure.

Weiterhin umfaßt die Wirkstoffkombination im Sinne der Erfindung als Wirkstoffkomponente ii) Vitamin B6, ein physiologisch akzeptables Derivat und/oder Salz davon. Gemische dieser Formen sind ebenfalls möglich, jedoch nur in bestimmten Fällen in Betracht zu ziehen. Gemäß einer besonderen Ausführungsform besteht die Wirkstoffkomponente ii) aus wenigstens 90 Gew.-% Pyridoxin-HCl.

Weiterhin umfaßt die Wirkstoffkombination im Sinne der Erfindung als Wirkstoffkomponente iii) Vitamin B12, ein physiologisch akzeptables Derivat und/oder Salz davon. Gemische dieser Formen sind ebenfalls möglich, jedoch nur in bestimmten Fällen in Betracht zu ziehen. Gemäß einer besonderen Ausführungsform besteht die Wirkstoffkomponente iii) aus wenigstens 90 Gew.-% Cobalamin.

Der Anteil der Wirkstoffkombination an der Formulierung ist größer als ein gegebenenfalls in natürlichen Quellen, insbesondere Lebensmitteln, vorhandener Anteil. In diesem Sinne sind die erfindungsgemäßen Mittel im Hinblick auf die Wirkstoffkombination angereichert. Der Anteil der Wirkstoffkombination aus i), ii) und iii) an der Formulierung beträgt vorzugsweise mindestens etwa 0,01 Gew.-%, vorteilhafterweise mindestens etwa 0,05 Gew.-% und insbesondere mindestens etwa 0,1 Gew.-%. Im Falle eines pharmazeutischen Mittels liegt der Anteil in der Regel bei etwa 1 bis 60 Gew.-%, vorzugsweise bei etwa 5 bis 35 Gew.-% und insbesondere bei etwa 10 bis 30 Gew.-%, im Falle eines Nahrungsergänzungsmittels und vor allem bei Lebensmitteln gegebenenfalls entsprechend niedriger, wenn die Formulierung in größeren Mengen zugeführt wird. Vorzugsweise enthalten die Formulierungen die angegebene Tagesdosis.

Angaben in Gew.-% beziehen sich, sofern nicht anderes angegeben ist, auf das Gesamtgewicht der Formulierung.

Die Formulierungsgrundlage erfindungsgemäßer pharmazeutischer Formulierungen enthält physiologisch akzeptable Hilfsstoffe. Physiologisch akzeptabel sind die im Bereich der Pharmazie, der Lebensmitteltechnologie und angrenzenden Gebieten bekanntermaßen verwendbaren Hilfstoffe, insbesondere die in einschlägigen Arzneibüchern (z.B. DAB, Ph. Eur., BP, NF) gelisteten, und auch andere Hilfstoffe, deren Eigenschaften einer physiologischen Anwendung nicht entgegenstehen. Hilfsstoffe im erfindungsgemäßen Sinne können auch einen Nährwert besitzen und deshalb allgemein als Nahrungskomponente verwendet werden. Auch Nährstoffe, insbesondere essentielle Nährstoffe, können dazu gehören.

Geeignete Hilfsstoffe können sein: Netzmittel; emulgierende und suspendierende Mittel; konservierende Mittel; Antioxidantien; Antireizstoffe; Chelatbildner; Dragierhilfsmittel; Emulsionsstabilisatoren; Filmbildner; Gelbildner; Geruchsmaskierungsmittel; Geschmackskorrigentien; Harze; Hydrokolloide; Lösemittel; Lösungsvermittler; Neutralisierungsmittel; Permeationsbeschleuniger; Pigmente; quaternäre Ammoniumverbindungen; Rückfettungs- und Überfettungsmittel; Salben-, Creme- oder Öl-Grundstoffe; Silikon-Derivate; Spreithilfsmittel; Stabilisatoren; Sterilanzien; Suppositoriengrundlagen; Tabletten-Hilfsstoffe, wie Bindemittel, Füllstoffe, Gleitmittel, Sprengmittel oder Überzüge; Treibmittel; Trocknungsmittel; Trübungsmittel; Verdickungsmittel; Wachse; Weichmacher; Weißöle. Eine diesbezügliche Ausgestaltung beruht auf fachmännischem Wissen, wie beispielsweise in Fiedler, H.P., Lexikon der Hilfsstoffe für Pharmazie, Kosmetik und angrenzende Gebiete, 4. Auflage, Aulendorf: ECV-Editio-Cantor-Verlag, 1996, dargestellt ist.

Nahrungskomponenten enthalten in der Regel eine oder mehrere Aminosäuren, Kohlenhydrate oder Fette und sind für die menschliche und/oder tierische Ernährung geeignet. Sie umfassen Einzelkomponenten, häufig pflanzliche aber auch tierische Produkte, insbesondere Zucker gegebenenfalls in Form von Sirups, Fruchtzubereitungen, wie Fruchtsäfte, Nektar, Fruchtpulpen, Pürees oder getrocknete Früchte, beispielsweise Apfelsaft, Grapefruitsaft, Orangensaft, Apfelmus, Tomatensauce, Tomatensaft, Tomatenpüree; Getreideprodukte, wie Weizenmehl, Roggenmehl, Hafermehl, Maismehl, Gerstenmehl, Dinkelmehl, Maissirup, sowie Stärken der genannten Getreide; Milchprodukte, wie Milcheiweiß, Molke, Joghurt, Lecithin und Milchzucker.

Zu den essentiellen Nährstoffen zählen insbesondere Vitamine, Provitamine, Spurenelemente, Aminosäuren und Fettsäuren. Als essentielle Aminosäuren seien genannt Isoleucin, Leucin, Lysin, Methionin, Phenylalanin, Threonin, Tryptophan und Valin. Dazu gehören auch semi-essentielle Aminosäuren, die beispielsweise in Wachstumsphasen oder Mangelzuständen zugeführt werden müssen, wie Arginin, Histidin, Cystein und Tyrosin. Als Spurenelemente seien genannt: essentielle Spurenelemente, deren Notwendigkeit für den Menschen erwiesen ist und deren Mangel zur Manifestation klinischer Symptome führt: Eisen, Kupfer, Zink, Chrom, Selen, Calcium, Magnesium, Kalium, Lithium, Cobalt, Molybdän, Iod, Silicium, Fluor, Mangan. Ebenso Elemente, deren Funktion für den Menschen noch nicht genügend gesichert ist: Zinn, Nickel, Vanadium, Arsen, Mangan. Als für den Menschen essentielle Fettsäuren seien genannt: Linolsäure und Linolensäure. Eine umfassende Aufzählung von Vitaminen findet sich in "Referenzwerte für die Nährstoffzufuhr", 1. Auflage, Umschau Braus Verlag, Frankfurt am Main, 2000, herausgegeben von der Deutschen Gesellschaft für Ernährung.

Die Summe aus Wirkstoffkomponente und Formulierungsgrundlage beträgt in der Regel 100 Gew.-%.

Beispiele geeigneter Formulierungen zur Nahrungsergänzung sind Kapseln, Tabletten, Pillen, Pulverbeutel, Flüssigampullen und Fläschchen mit Tropfeinsätzen, im übrigen die nachfolgend genannten Arzneiformen.

Beispiele geeigneter pharmazeutischer Formulierungen sind feste Arzneiformen, wie Pulver, Puder, Granulate, Tabletten, insbesondere Filmtabletten, Pastillen, Sachets, Cachets, Dragees, Kapseln wie Hart- und Weichgelatinekapseln, Suppositorien oder vaginale Arzneiformen, halbfeste Arzneiformen, wie Salben, Cremes, Hydrogele, Pasten oder Pflaster, sowie flüssige Arzneiformen, wie Lösungen, Emulsionen, insbesondere Öl-in-Wasser-Emulsionen, Suspensionen, beispielsweise Lotionen, Injektions- und Infusionszubereitungen, Augen- und Ohrentropfen. Auch implantierte Abgabevorrichtungen können zur Verabreichung erfindungsgemäßer Wirkstoffe verwendet werden. Ferner können auch Liposomen oder Mikrosphären zur Anwendung kommen.

Lebensmitteltechnische Formulierungen haben in der Regel die übliche Form und werden bevorzugt in Form von Kleinkindnahrung, Frühstückszubereitungen, vor allem in Form von Müslis oder Riegeln, Sportlerdrinks, Komplettmahlzeiten, insbesondere im Rahmen von total bilanzierten Diäten, diätetische Zubereitungen, wie Diätdrinks, Diätmahlzeiten und Diätriegel, angeboten.

Die Formulierungen werden vorzugsweise auf oralem, sie können aber auch insbesondere im Breich der Arzneimittel auf rektalem, transdermalem, subkutanem, intravenösem, intramuskulärem oder intranasalem Weg verabreicht werden.

Bei der Herstellung der Zusammensetzungen werden die Wirkstoffe gewöhnlich mit einem geeigneten Hilfsstoff, in diesem Fall auch als Exzipient zu bezeichnen, vermischt oder verdünnt. Exzipienten können feste, halbfeste oder flüssige Materialien sein, die als Vehikel, Träger oder Medium für den Wirkstoff dienen. Die Zumischung weiterer Hilfsstoffe erfolgt erforderlichenfalls in an sich bekannter Weise. Es können Formgebungsschritte, gegebenenfalls in Verbindung mit Mischvorgängen, durchgeführt werden, z.B. eine Granulierung, Komprimierung und ähnliches.

Insbesondere können die Wirkstoffkomponenten gemeinsam formuliert werden. Sie können aber auch zunächst getrennt verarbeitet und anschließend in einer kompartimentierten, z.B. mehrschichtigen Arzneiform zusammengeführt werden. Dadurch kann möglichen Wirkstoffinkompatibilitäten und unterschiedlichen Wirkstoffeigenschaften, wie Bioverfügbarkeit, Stabilität, Löslichkeit und ähnlichem, Rechnung getragen werden.

Die vorliegende Erfindung wird anhand der nachfolgenden Beispiele näher erläutert, ohne darauf beschränkt zu sein.

### Beispiel 1

### Pharmazeutische Mittel

### a) Weichgelatine-Kapsel

### (Folsäure 1 mg + Vitamin B6 50 mg + Vitamin B12 1 mg)

### Füllung:

| | | |
|---|---|---|
| Folsäure | 1 | mg |
| Vitamin B6 | 50 | mg |
| Vitamin B12 | 1 | mg |
| Sojaöl (raff.) | 440 | mg |
| Sojalecithin (E322) | 50 | mg |
| Hochdisperses Siliciumdioxid | 5 | mg |

| Kapselhülle: | | |
|---|---|---|
| Gelatine | 303 | mg |
| Glycerol 85 % | 87 | mg |
| Sorbitol 70 % | 77 | mg |
| gereinigtes Wasser | 52 | mg |
| Eisenoxidpigment Braun 75 ( E 172) | 3 | mg |

### b) Tablette

### (Folsäure 1 mg + Vitamin B6 50 mg + Vitamin B12 1 mg)

| | | |
|---|---|---|
| Folsäure | 1 | mg |
| Vitamin B6 | 50 | mg |
| Vitamin B12 | 1 | mg |
| Milchzucker | 127,5 | mg |
| Magnesiumstearat | 5 | mg |
| Talcum | 23,75 | mg |
| Mikrokristalline Cellulose | 81 | mg |

### Beispiel 2

### Patient nach Myokardinfarkt

Der Homocysteinwert eines 42-jährigen Mannes nach Myokardinfarkt betrug 39,3 µmol/l. Verabreichte man diesem Mann täglich oral 1 mg Folsäure, 1 mg Vitamin B12 und 100 mg Vitamin B6, sank der Homocysteinwert nach 8 Wochen auf 12,1 µmol/l ab. Eine weitere Senkung trat im Verlauf der sich anschließenden 3 Monate trotz fortgesetzter Therapie nicht ein. Setzte man die Therapie ab, stieg der Homocysteinwert innerhalb von 8 Wochen erneut auf 39,3 µmol/l an.

Verabreichte man nun täglich oral 1 mg Folsäure, 1 mg Vitamin B12 und 50 mg Vitamin B6, sank der Homocysteinwert nach 8-wöchiger Therapie auf 14,6 µmol/l ab.

Weitere Therapieversuche mit abweichenden Mengenverhältnissen der drei Wirkstoffe führten zu einer vergleichsweise geringeren Senkung des Homocysteinwerts. So sank der Homocysteinwert lediglich auf 34,5 µmol/l, wenn man täglich oral 1 mg Folsäure, 1 mg Vitamin B12 und 75 mg Vitamin B6 verabreichte.

### Beispiel 3

### Patient mit insulinpflichtigem Diabetes mellitus, Ulcus cruris bei Nikotonabusus

Der Homocysteinwert eines 39-jährigen Patienten mit insulinpflichtigem Diabetes mellitus, Ulcus cruris bei Nikotonabusus betrug 49,1 µmol/l.

Verabreichte man diesem Mann täglich oral 1 mg Folsäure, 1 mg Vitamin B12 und 100 mg Vitamin B6 sank der Homocysteinspiegel auf 16,3 µmol/l ab. Allerdings trat bereits an Tag 8 nach Therapiebeginn wiederholt ein Kribbeln an den Händen, Armen und Beinen des Pateinten auf. Setzte man die Therapie aus, stieg der Homocysteinspiegel nach 4 Wochen erneut auf 49,2 µmol/l an.

Eine anschließende Therapie mit täglicher oraler Verabreichung von 1 mg Folsäure, 1 mg Vitamin B12 und 50 mg Vitamin B6 führte nach 8 Wochen zu einem Absinken des Homocysteinspiegels auf 18,2 µmol/l. Die zuvor beobachteten Nebenwirkungen traten hier nicht auf.

Setzte man diese Therapie ab, stieg der Homocysteinwert nach 8 Wochen erneut auf 49,2 µmol/l an.

## Patentansprüche

1. Mittel auf Basis von Folsäure, Vitamin B6 und Vitamin B12 beziehungsweise physiologisch akzeptabler Derivate und/oder Salze davon, **dadurch gekennzeichnet, dass** die Gewichtsmengenverhältnisse von Folsäure : Vitamin B6 und von Vitamin B12 : Vitamin B6 in einem Bereich von 1 : 41-61, sowie von Folsäure : Vitamin B12 in einem Bereich von 1 : 0,82-1,22 liegen,
wobei die physiologisch akzeptablen Folsäurederivate ausgewählt sind unter Folsäuremetaboliten der Formel Ib 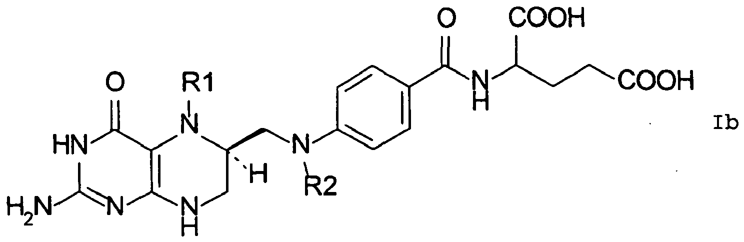 worin R1 für Wasserstoff, Methyl, -HC=O oder -HC=NH steht und R2 für Wasserstoff oder -HC=O steht, oder R1 und R2 zusammen genommen eine Methylen- oder Methenylbrücke bilden,
sowie Estern und Amiden der Folsäure und der Folsäure-metabolite,
die physiologisch akzeptablen Vitamin B6-Derivate ausgewählt sind unter Pyridoxinen, Pyridoxalen und Pyridoxaminen der Formel IIa 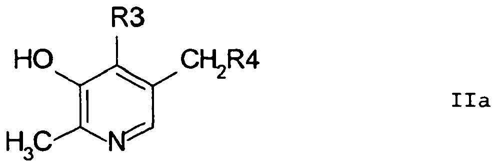 worin R3 für CH₂OH, CHO oder CH₂NH₂ steht und R4 für OH oder OPO₃H₂ steht,
sowie Estern der Pyridoxine, Pyridoxale und Pyridoxamine und
die physiologisch akzeptablen Vitamin B12-Derivate ausgewählt sind unter Cobalaminen, in denen die Cyanogruppe des Cyanocobalamins durch andere Koordinationspartner des Cobalts ersetzt ist.

2. Mittel nach Anspruch 1, **dadurch gekennzeichnet, dass** das Gewichtsmengenverhältnis von Folsäure : Vitamin B6 : Vitamin B12 etwa 1 : 50 : 1 beträgt.

3. Verwendung von Folsäure, Vitamin B6 und Vitamin B12 beziehungsweise wie in Anspruch 1 definierter physiologisch akzeptabler Derivate und/oder Salze davon zur Herstellung eines Mittels zur Regulation von Homocysteinspiegeln, **dadurch gekennzeichnet, dass** die Gewichtsmengenverhältnisse von Folsäure : Vitamin B6 und von Vitamin B12 : Vitamin B6 in einem Bereich von 1 : 33-75, sowie von Folsäure : Vitamin B12 in einem Bereich von 1 : 0,67-1,50 liegen, wobei die Tagesdosis weniger als 70 mg Vitamin B6 beträgt.

4. Verwendung von Folsäure, Vitamin B6 und Vitamin B12 beziehungsweise wie in Anspruch 1 definierter physiologisch akzeptabler Derivate und/oder Salze davon zur Herstellung eines Mittels zur Behandlung vaskulärer Erkrankungen, **dadurch gekennzeichnet, dass** die Gewichtsmengenverhältnisse von Folsäure : Vitamin B6 und von Vitamin B12 : Vitamin B6 in einem Bereich von 1 : 33-75, sowie von Folsäure : Vitamin B12 in einem Bereich von 1 : 0,67-1,50 liegen, wobei die Tagesdosis weniger als 70 mg Vitamin B6 beträgt.

5. Verwendung nach Anspruch 4, wobei die vaskuläre Erkrankung eine Arteriosklerose, ein arterieller Verschluss, eine venöse Thrombose oder eine vaskulär bedingte Form der Altersdemenz ist.

6. Verwendung von Folsäure, Vitamin B6 und Vitamin B12 beziehungsweise wie in Anspruch 1 definierter physiologisch akzeptabler Derivate und/oder Salze davon zur Herstellung eines Mittels zur Behandlung von schwangeren Frauen, **dadurch gekennzeichnet, dass** die Gewichtsmengenverhältnisse von Folsäure : Vitamin B6 und von Vitamin B12 : Vitamin B6 in einem Bereich von 1 : 33-75, sowie von Folsäure : Vitamin B12 in einem Bereich von 1 : 0,67-1,50 liegen, wobei die Tagesdosis weniger als 70 mg Vitamin B6 beträgt.

7. Verwendung nach einem der Ansprüche 4 bis 6, wobei die Tagesdosis 0,8 mg bis 1,2 mg Folsäure, 40 mg bis 60 mg Vitamin B6 sowie 0,8 mg bis 1,2 mg Vitamin B12 beträgt.

8. Verwendung nach einem der Ansprüche 4 bis 6, wobei die Tagesdosis 0,9 mg bis 1,1 mg Folsäure, 45 mg bis 55 mg Vitamin B6 sowie 0,9 mg bis 1,1 mg Vitamin B12 beträgt.

9. Verwendung nach einem der Ansprüche 4bis 6, wobei die Tagesdosis 1 mg Folsäure, 50 mg Vitamin B6 sowie 1 mg Vitamin B12 beträgt.

## Claims

1. Agent based on folic acid, vitamin B6 and vitamin B12 or physiologically acceptable derivatives and/or salts thereof, **characterised in that** the weight ratios of folic acid: vitamin B6 and of vitamin B12: vitamin B6 are in a range from 1 : 41-61, and those of folic acid : vitamin B12 are in a range from 1 : 0.82 -1.22,
whereby the physiologically acceptable folic acid derivatives are selected from among folic acid metabolites of formula Ib 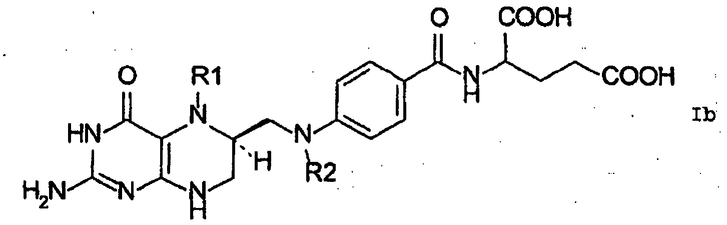 wherein R1 denotes hydrogen, methyl, -HC=O or-HC=NH and R2 denotes hydrogen or -HC=O, or R1 and R2 taken together form a methylene or methenyl bridge, and esters and amides of folic acid and the folic acid metabolites,
the physiologically acceptable vitamin B6 derivatives are selected from among pyridoxines, pyridoxals and pyridoxamines of formula IIa 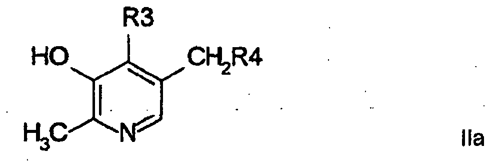 wherein R3 denotes CH₂OH, CHO or CH₂NH₂ and R4 denotes OH or OPO₃H₂, and esters of the pyridoxines, pyridoxals and pyridoxamines and
the physiologically acceptable vitamin B12 derivatives selected from among cobalamins, wherein the cyano group of the cyanocobalamin is replaced by other coordination partners of cobalt.

2. Agent according to claim 1, **characterised in that** the ratio by weight of folic acid : vitamin B6 : vitamin B12 is about 1 : 50 : 1.

3. Use of folic acid, vitamin B6 and vitamin B12 or physiologically acceptable derivatives and/or salts thereof as defined in claim 1, for preparing an agent for the regulation of homocysteine levels, **characterised in that** the ratios by weight of folic acid : vitamin B6 and of vitamin B12 : vitamin B6 are in a range from 1 : 33-75, and those of folic acid : vitamin B12 are in a range from 1 : 0.67-1.50, the daily dose being less than 70 mg of vitamin B6.

4. Use of folic acid, vitamin B6 and vitamin B12 or physiologically acceptable derivatives and/or salts thereof as defined in claim 1, for preparing an agent for the treatment of vascular diseases, **characterised in that** the ratios by weight of folic acid : vitamin B6 and of vitamin B12 : vitamin B6 are in a range from 1 : 33-75, and those of folic acid : vitamin B12 are in a range from 1 : 0.67-1.50, the daily dose being less than 70 mg of vitamin B6.

5. Use according to claim 4, wherein the vascular disease is arteriosclerosis, arterial blockage, venous thrombosis or a form of senile dementia of vascular origin.

6. Use of folic acid, vitamin B6 and vitamin B12 or physiologically acceptable derivatives and/or salts thereof as defined in claim 1, for preparing an agent for the treatment of pregnant women, **characterised in that** the ratios by weight of folic acid : vitamin B6 and of vitamin B12 : vitamin B6 are in a range from 1 : 33-75, and those of folic acid : vitamin B12 are in a range from 1 : 0.67-1.50, the daily dose being less than 70 mg of vitamin B6.

7. Use according to one of claims 4 to 6, wherein the daily dose is 0.8 mg to 1.2 mg of folic acid, 40 mg to 60 mg of vitamin B6 and 0.8 mg to 1.2 mg of vitamin B12.

8. Use according to one of claims 4 to 6, wherein the daily dose is 0.9 mg to 1.1 mg of folic acid, 45 mg to 55 mg of vitamin B6 and 0.9 mg to 1.1 mg of vitamin B12.

9. Use according to one of claims 4 to 6, wherein the daily dose is 1 mg of folic acid, 50 mg of vitamin B6 and 1 mg of vitamin B12.

## Revendications

1. Produit à base d'acide folique, de vitamine B6 et de vitamine B12, ou de dérivés et/ou sels physiologiquement acceptables de ces derniers, **caractérisés en ce que** les proportions pondérales acide folique:vitamine B6 et vitamine B12:vitamine B6 sont comprises dans la plage de 1:41-61, et la proportion pondérale acide folique:vitamine B12 est comprise dans la plage de 1:0,82-1,22,
dans lequel les dérivés de l'acide folique physiologiquement acceptables sont choisis parmi les métabolites de l'acide folique de formule Ib 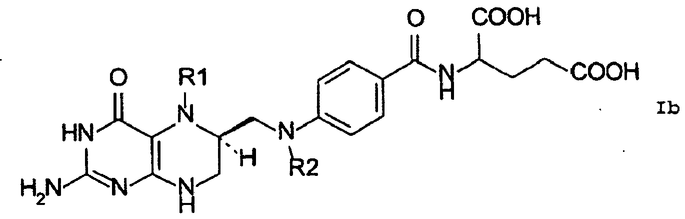 dans laquelle R1 est un atome d'hydrogène, le groupe méthyle, -HC=O ou -HC=NH, et R2 est un atome d'hydrogène ou -HC=O, ou R1 et R2, pris ensemble, forment un pont méthylène ou méthényle, ainsi que les esters et amides de l'acide folique et les métabolites de l'acide folique,
les dérivés de la vitamine B6 physiologiquement acceptables sont choisis parmi les pyridoxines, les pyridoxals et les pyridoxamines de formule IIa 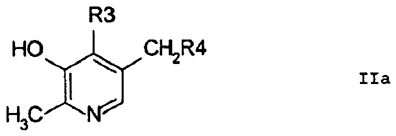 dans laquelle R3 est CH₂OH, CHO ou CH₂NH₂, et R4 est OH ou OPO₃H₂,
ainsi que les esters des pyridoxines, des pyridoxals et des pyridoxamines, et
les dérivés de la vitamine B12 physiologiquement acceptables sont choisis parmi les cobalamines dans lesquelles le groupe cyano de la cyanocobalamine est remplacé par d'autres partenaires de coordination du cobalt.

2. Produit selon la revendication 1, **caractérisé en ce que** la proportion pondérale acide folique:vitamine B6:vitamine B12 est d'environ 1:50:1.

3. Utilisation d'acide folique, de vitamine B6 et de vitamine B12, ou de dérivés et/ou sels de ces derniers, physiologiquement acceptables et définis comme dans la revendication 1, pour préparer un produit destiné à la régulation des niveaux d'homocystéine, **caractérisée en ce que** les proportions pondérales acide folique:vitamine B6 et vitamine B12:vitamine B6 sont comprises dans la plage de 1:33-75, et la proportion pondérale acide folique:vitamine B12 est comprise dans la plage de 1:0,67-1,50, la dose journalière étant inférieure à 70 mg de vitamine B6.

4. Utilisation d'acide folique, de vitamine B6 et de vitamine B12, ou de dérivés et/ou sels de ces derniers, physiologiquement acceptables et définis comme dans la revendication 1, pour préparer un produit destiné au traitement de maladies vasculaires, **caractérisée en ce que** les proportions pondérales acide folique:vitamine B6 et vitamine B12:vitamine B6 sont comprises dans la plage de 1:33-75, et la proportion pondérale acide folique:vitamine B12 est comprise dans la plage de 1:0,67-1,50, la dose journalière étant inférieure à 70 mg de vitamine B6.

5. Utilisation selon la revendication 4, pour laquelle la maladie vasculaire est une artériosclérose, une occlusion artérielle, une thrombose veineuse ou une forme d'origine vasculaire de la démence sénile.

6. Utilisation d'acide folique, de vitamine B6 et de vitamine B12, ou de dérivés et/ou sels de ces derniers, physiologiquement acceptables, définis comme dans la revendication 1, pour préparer un produit destiné au traitement des femmes enceintes, **caractérisée en ce que** les proportions pondérales acide folique:vitamine B6 et vitamine B12:vitamine B6 sont comprises dans la plage de 1:33-75, et la proportion pondérale acide folique:vitamine B12 est comprise dans la plage de 1:0,67-1,50, la dose journalière étant inférieure à 70 mg de vitamine B6.

7. Utilisation selon l'une des revendications 4 à 6, **caractérisée en ce que** la dose journalière est de 0,8 mg à 1,2 mg d'acide folique, de 40 mg à 60 mg de vitamine B6, ainsi que de 0,8 mg à 1,2 mg de vitamine B12.

8. Utilisation selon l'une des revendications 4 à 6, pour laquelle la dose journalière est de 0,9 mg à 1,1 mg d'acide folique, de 45 mg à 55 mg de vitamine B6, ainsi que de 0,9 mg à 1,1 mg de vitamine B12.

9. Utilisation selon l'une des revendications 4 à 6, pour laquelle la dose journalière est de 1 mg d'acide folique, de 50 mg de vitamine B6, ainsi que de 1 mg de vitamine B12.
